# EUROPEAN PATENT APPLICATION

(11) **EP 4 653 866 A1**
(43) Date of publication of application: **26.11.2025**
(21) Application number: 25178314.8
(22) Date of filing: 22.05.2025
(51) Int. Cl.: G01N 33/00

(54) **SYSTEM AND METHOD FOR MEASURING A GAS CONCENTRATION IN ONE OR MORE REACTION CHAMBERS**

(30) Priority: 23.05.2024 US 202418673069
(71) Applicant: Viavi Solutions Inc., Chandler, AZ 85286 (US)
(72) Inventor: JANSSEN, Kelly, Santa Rosa, CA 95407 (US); THORAVAL, Carole, Santa Rosa, CA 95405 (US); DAVIES, Barbara, Rohnert Park, CA 94928 (US); NOFI, Michael R., Rohnert Park, CA 94928 (US)
(74) Representative: Murgitroyd & Company

(57) **Abstract**

A system for determining a concentration of a gas in one or more reaction chambers. The system comprises an energy source configured to provide a reaction energy to the one or more reaction chambers. A gas meter is capable of measuring a concentration of a gas in the one or more reaction chambers. A method for determining a concentration of a gaseous product in one or more reaction chambers is also disclosed. A test kit and a reaction chamber are also disclosed.

## Description

### FIELD OF THE DISCLOSURE

The present disclosure is directed to a system and method for determining a concentration of a gas, such as a gas product produced by a chemical reaction, in one or more reaction chambers.

### BACKGROUND

Some products, such as pigments used in paints and other compositions, can potentially react with their environment to form unwanted gases. It is known in the art to test such products for gassing stability. One conventional test is a waterborne paint gassing stability test that involves mixing passivated aluminum containing pigments in water-based paint and allowing the mixture to sit for up to 28-days. The test is set up to measure a volume displacement of gasses generated during the test, and assumes that all volume displacement is due to hydrogen gas production caused by reaction of water with the aluminum in the pigment. This test is used as an industry standard for testing passivated pigments.

The above waterborne paint gassing stability test is problematic in that the time required to test samples is extensive (e.g., taking several weeks), the test setup is cumbersome, and the test is labor intensive. Further, the volume displacement method assumes all volume changes are due to hydrogen gas production, which may or may not be correct, potentially resulting in incorrect measurements for hydrogen outgassing. Additionally, the method only works for powders and pigments, not other media, and requires the use of binders and other components that may involve different chemistries specific to customers and that may produce different results. Further, industry-standard test samples are not always comparable from test to test due to the influence of atmospheric conditions.

What is needed are novel systems and methods that can solve one or more of the above problems, such as by providing relatively quick methods to test reactive products for potential out-gassing, and/or the ability to compare out-gassing of test products or materials used to make products, for example, metal powders used for making pigments or other products prior to passivation, as well as for testing films and other items vulnerable to reactive processes like corrosion.

### BRIEF DESCRIPTION OF THE DRAWINGS

Features of the present disclosure are illustrated by way of example and not limited in the following figure(s), in which like numerals indicate like elements, in which:
FIG. 1 is a block diagram of a system for determining a concentration of gas product in one or more reaction chambers, according to an embodiment of the present disclosure.
FIG. 2 illustrates a schematic diagram of a system for determining a concentration of gas product in one or more reaction chambers, according to an embodiment of the present disclosure.
FIG. 3 illustrates a schematic view of a reaction chamber, according to an embodiment of the present disclosure.
FIG. 4A illustrates a perspective view of a volume control inset, according to an embodiment of the present disclosure.
FIG. 4B illustrates a perspective view of a volume control inset being inserted into a reaction chamber, according to an embodiment of the present disclosure.
FIGS. 5A, 5B, and 5C illustrate various perspective views of a fixture for holding a reaction chamber, according to an embodiment of the present disclosure.
FIG. 6 illustrates a schematic view of a system comprising an auto-sampler, according to an embodiment of the present disclosure.
FIGS. 7A and 7B each illustrate different views of a tab configured to cover or uncover a perforation in the reaction chamber of FIG. 3, according to an embodiment of the present disclosure.
FIG. 8 illustrates a schematic view of a connector device for fluidly connecting a gas meter to the inner volume of a reaction chamber, according to an embodiment of the present disclosure.

### SUMMARY

In an aspect, there is disclosed a system for determining a concentration of a gas in one or more reaction chambers, the system comprising: an energy source configured to provide a reaction energy to the one or more reaction chambers; and a gas meter capable of measuring a concentration of a gas in the one or more reaction chambers.

The gas meter may be a hydrogen gas meter, oxygen gas meter, carbon monoxide gas meter, carbon dioxide gas meter or a chlorine gas meter.

The system may further comprise a fixture configured to hold at least one of the one or more reaction chambers.

The system may further comprise an auto-sampler configured to convey the one or more reaction chambers to a reaction site.

The system may further comprise a connector device fluidly connected to the gas meter, the connector device configured to fluidly connect the gas meter with an inner volume of the one or more reaction chambers.

The system may further comprise a computer that includes a non-transitory computer readable medium, the non-transitory computer readable medium may comprise computer readable instructions for collecting and storing a gas concentration transmitted from the gas meter for each reaction chamber tested and providing an output associating the gas concentration with a sample contained in the reaction chamber from which it was collected.

In an aspect, there is disclosed a method for determining a concentration of a gaseous product in one or more reaction chambers, the method comprising: hermetically sealing an exposure reactant and a test product comprising a test product reactant in one or more reaction chambers; exposing the one or more reaction chambers to ambient conditions sufficient to cause the exposure reactant and the test product reactant to react to form a gaseous product if the test product reactant is available for reaction with the exposure reactant; and determining the concentration of the gaseous product in the one or more reaction chambers.

The test product reactant may be a metal chosen from aluminum, iron, copper, magnesium, zinc, manganese, silver, platinum, palladium, tungsten, nickel, cobalt, niobium, chromium, tin, titanium and alloys thereof.

The exposure reactant may be chosen from water and an acid.

The gaseous product may be a compound chosen from hydrogen gas, oxygen gas, carbon dioxide, carbon monoxide and chlorine gas.

The test product may be a pigment, a foil, a coating, a filler powder, a nanowire or a catalyst.

The method may further comprise carrying out the processes of hermetically sealing, exposing the reaction chamber and determining the concentration of the gaseous product for a plurality of test products in two or more reaction chambers, where each test product is contained in a separate reaction chamber of the two or more reaction chambers.

The exposing of the one or more reaction chambers to ambient conditions sufficient to cause the exposure reactant and the test product reactant to react may be carried out for a time period that ranges from about 1 hour to about 1 week.

The one or more reaction chambers may each have a variable volume.

The method may further comprise inserting a volume control inset into each of the one or more reaction chambers.

The one or more reaction chambers may each comprise a metalized bag.

The method may further comprise inserting at least one reactant reservoir into the one or more reaction chambers, wherein the at least one reactant reservoir may be gas permeable.

In a further aspect, there is disclosed a test kit having two or more components, the kit comprising: one or more reaction chambers, each reaction chamber comprising (i) a gas impermeable material enclosing an inner volume and (ii) a hermetically sealable opening; at least one reactant reservoir configured to be inserted into the one or more reaction chambers; and a volume control inset configured to be inserted into each of the one or more reaction chambers so as to provide each container with a desired volume.

The test kit may further comprise a gas meter capable of measuring a concentration of a gas in the one or more reaction chambers; and optionally a fixture configured to hold the one or more reaction chambers while measuring the concentration of gas.

In as aspect, there is disclosed a reaction chamber, comprising: a gas impermeable material at least partially enclosing an inner volume; a hermetically sealable opening; and a hermetically sealable port disposed in the gas impermeable material, wherein the reaction chamber has a variable inner volume.

The systems and methods of the present application can provide one or more of the following advantages: reducing the amount of time required to assess the effectiveness of corrosion inhibition processes on aluminum-based pigments; reducing sample preparation time and effort; a relatively less cumbersome test setup requiring relatively small space; the ability to directly measure hydrogen without, for example, measuring volume displacement; testing that is not dependent on a particular binder chemistry, such as a paint system; ability to provide relative accuracy, sensitivity and/or precision compared with traditional volume displacement methods for measuring hydrogen formation; the ability to allow for precise control of conditions that lead to accelerated formation of hydrogen, such as an amount of water accessing the metal powders being tested, system temperature and presence of any reaction catalysts; the ability to adapt process conditions to achieve the range of desired hydrogen concentrations for improving the accuracy of the hydrogen sensor used; the ability to provide pre-screening methods not provided by existing technologies for pigments, such as aluminum-based pigments, to assess their quality and suitability for the passivation process prior to passivation; the ability to make relatively quick sample-to-sample comparisons possible; the ability to make sample-to-sample comparisons possible without, or with reduced, interference from atmospheric condition fluctuations, such as those seen in current industry standard volume displacement testing; the capability to be used for testing of a wide range of applications, including pigment testing, as well as testing of non-pigment applications such as foils, coatings, filler powders, nanowires, catalyst, and so forth; and the ability to adapt passivation process conditions depending on the gassing properties of the aluminum-based pigments.

Additional features and advantages of various embodiments will be set forth, in part, in the description that follows, and will, in part, be apparent from the description, or can be learned by the practice of various embodiments. The objectives and other advantages of various embodiments will be realized and attained by means of the elements and combinations particularly pointed out in the description herein.

### DETAILED DESCRIPTION

For simplicity and illustrative purposes, the present disclosure is described by referring mainly to examples thereof. In the following description, numerous specific details are set forth in order to provide a thorough understanding of the present disclosure. It will be readily apparent however, that the present disclosure may be practiced without limitation to these specific details. In other instances, some methods and structures have not been described in detail so as not to unnecessarily obscure the present disclosure.

Additionally, the elements depicted in the accompanying figures may include additional components and some of the components described in those figures may be removed and/or modified without departing from the scope of the present disclosure. Further, the elements depicted in the figures may not be drawn to scale and thus, the elements may have sizes and/or configurations that differ from those shown in the figures.

It is to be understood that both the foregoing general description and the following detailed description are exemplary and explanatory only, and are intended to provide an explanation of various embodiments of the present teachings.

In its broad and varied embodiments, disclosed herein is a system 10 for determining a concentration of a gas in one or more reaction chambers. In an embodiment, the gas is a product of a reaction that occurred in the reaction chamber, although any gas in the one or more reaction chambers could potentially be measured. Optionally, the gas concentration determined can be used to compare a relative extent of reaction between at least two of the one or more reaction chambers.

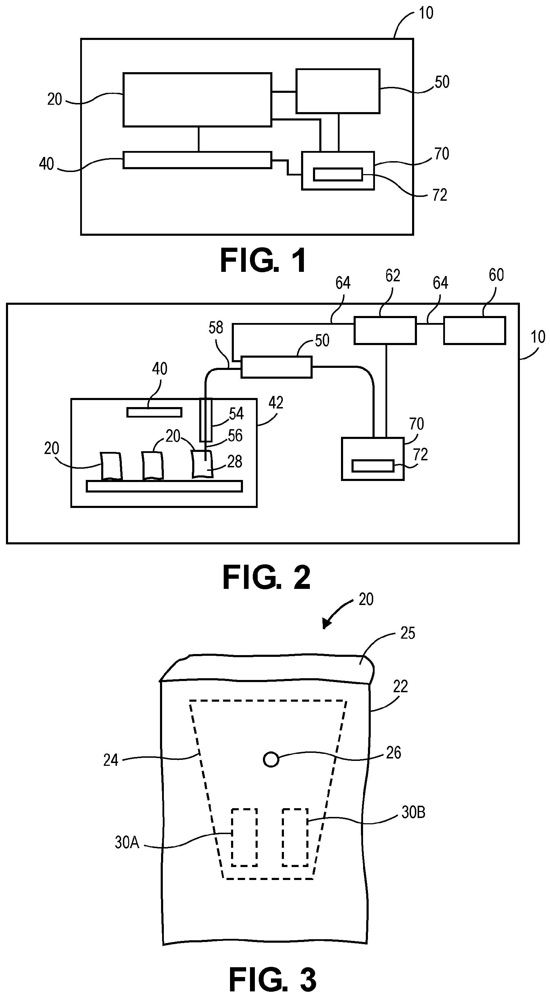

FIG. 1 is a block diagram of a system 10, according to an embodiment of the present disclosure. System 10 can comprise or be configured for processing one or more reaction chambers 20. An energy source 40 is configured to provide a reaction energy to the one or more reaction chambers 20. The system 10 further comprises a gas meter 50 capable of measuring a concentration of a gas in the one or more reaction chambers 20 and an optional computer 70. Various embodiments of the system 10 and/or the above mentioned components of system 10, including the one or more reaction chambers 20, energy source 40, gas meter 50 and computer 70, will be discussed in greater detail herein, and any of the embodiments or examples of the various components described herein can be incorporated as part of the system 10 of FIG. 1. Any two or more, such as all, of the above mentioned components of system 10 can be integrated together in a single apparatus. For example, the energy source 40 and gas meter 50 can be integrated together in a single apparatus. Alternatively, the energy source 40, gas meter 50 and the computer 70 can be integrated together in a single apparatus. In addition to the above described integrated components, one or more of any of the other components described herein below, such as a fixture 32, enclosure 42, conveyor 44, mechanical extender 54, connector device 56 and/or auto-sampler 80, can also optionally be integrated into a single apparatus of system 10. Additionally, any two or more, such as all, of the above mentioned components of system 10 can be electronically connected (e.g., hard-wired or wirelessly connected) to allow electronic communication (e.g., data transfer) between the components. For example, the energy source 40, the gas meter 50 or both, can be electronically connected to the computer 70. Thus, computer 70 can be employed to collect data from, and/or control, any or all of the components of system 10. For example, lines or arrows from the computer 70 to any of the various components in the figures of this application indicate that the components can be electronically connected to the computer 70 for purposes of data collection and/or process control.

FIG. 2 illustrates a schematic diagram of a system 10 that comprises or is configured for processing one or more reaction chambers 20, according to an embodiment of the present disclosure. Each reaction chamber 20 comprises: (i) a gas impermeable material enclosing an inner volume and (ii) a hermetically sealable or sealed opening 25 (FIG. 3). An energy source 40 is configured to provide a reaction energy to the one or more reaction chambers 20. The system further comprises a gas meter 50 capable of measuring a concentration of a gas in the one or more reaction chambers 20 and an optional computer 70, as will be discussed in greater detail below.

The reaction chambers 20 can have any desired form and can be disposable or reusable. In an embodiment, the reaction chambers 20 are portable and sized for easy handling by a user. Examples of suitable reaction chambers 20 include vials, flasks or test tubes or other rigid containers with hermetically sealable lids; and bags or other flexible containers that are hermetically sealable.

The one or more reaction chambers 20 can comprise one or more gas impermeable materials, including organic or inorganic materials, such as metals, glass, polymers, or combinations thereof. The gas impermeable materials can be selected to withstand reaction temperatures and other reaction conditions. The materials can provide the desired impermeability to air so as to allow for an air-tight container that can be hermetically sealed. The container can comprise rigid or flexible materials, such as flexible polymers, metalized polymers, or foils comprising metal. In an example, the one or more reaction chambers 20 comprise a metallized polymer bag. Examples of suitable polymers that can be metalized include polyester, polyethylene, polyimide (e.g., KAPTON^{™}), or a combination of two or more of these polymers, or any other polymer that can withstand reaction temperatures. The polymer bags can be metalized with any suitable metal, such as aluminum, so as to provide a metal layer that covers a surface of the bag (e.g., the entire outer surface). An example of commercially available metalized polymer bags is ULINE Dri-Shield Bags, available from Uline of Pleasant Prairie, Wisconsin. The Dri-Shields Bags comprise Polyester, polyethylene and aluminum.

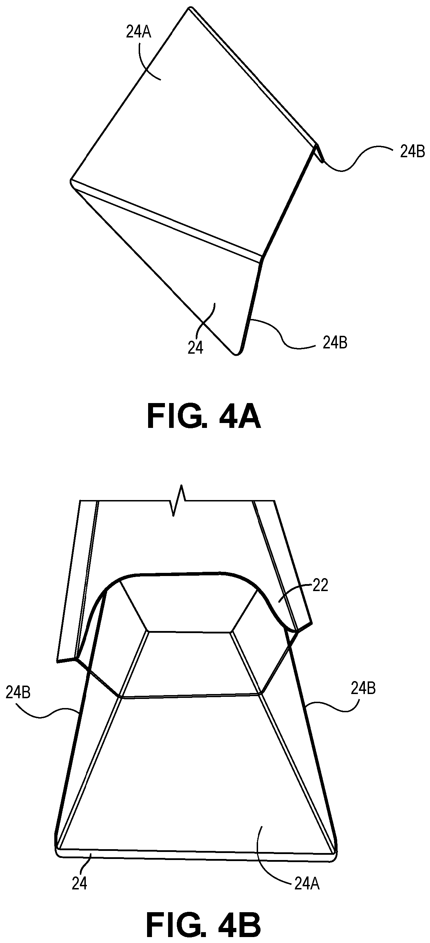

In an embodiment, the reaction chamber 20 can have an adjustable volume. For example, as illustrated in FIG. 3, the reaction chambers 20 can each comprise a hermetically sealable bag 22 and one or more volume control insets 24. The volume control insets 24 are configured to be inserted into each of the one or more hermetically sealable bags 22 so as to provide the hermetically sealable bag with an inner volume. The size of the inset can optionally be selected to provide an inner volume within a desired range. Optionally, insets of differing sizes and/or inserts that are configured to have adjustable sizes, can be included as part of the system or made available to a user. The size of the inset can then be chosen by a user to adjust the volume of the reaction chamber 20, as desired. An example of a volume control inset 24 is shown in FIGS. 4A, which includes a middle section 24A and two outer sections 24B positioned to extend at an angle from either side of the middle section so as to form an open space 24C between the outer sections 24B. Such a shape can be useful for maintaining a gas pocket within the reaction chambers 20 that can be useful when measuring the gas concentrations, as is discussed in more detail below. FIG. 4B shows the volume control inset 24 being inserted into a hermetically sealable bag 22. The insert can have any shape that is designed to maintain a desired volume of the bag while allowing the opening 25 of the hermetically sealable bag 22 to be sealed. The inset can comprise any material that can withstand reaction temperatures and provides the desired structural stability. Examples of suitable materials include polymers, such as polycarbonate, metals, glass (e.g., PYREX) or other materials.

The opening 25 can be configured to be hermetically sealed in any suitable manner. In an example, where a bag 22 is employed, the opening of the bag can be fused together using heat, such as by employing a heat sealing apparatus. Heat sealing apparatus are well known in the art. An example of a heat sealing apparatus is a HIPPO heat sealer, available from Impak Corporation of Los Angeles, California. Other techniques for sealing can also be used, such by using an adhesive capable of withstanding reaction temperatures. In other embodiments, a lid configured to hermetically seal the opening can be employed, such as in the case where rigid containers that are suitable for use with such lids can be employed.

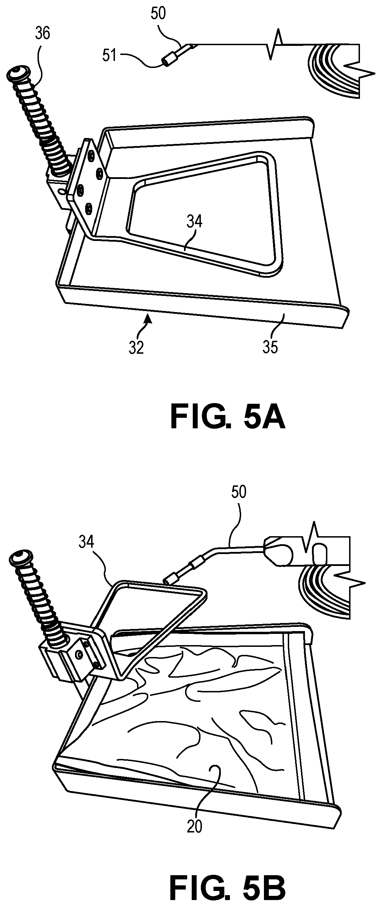

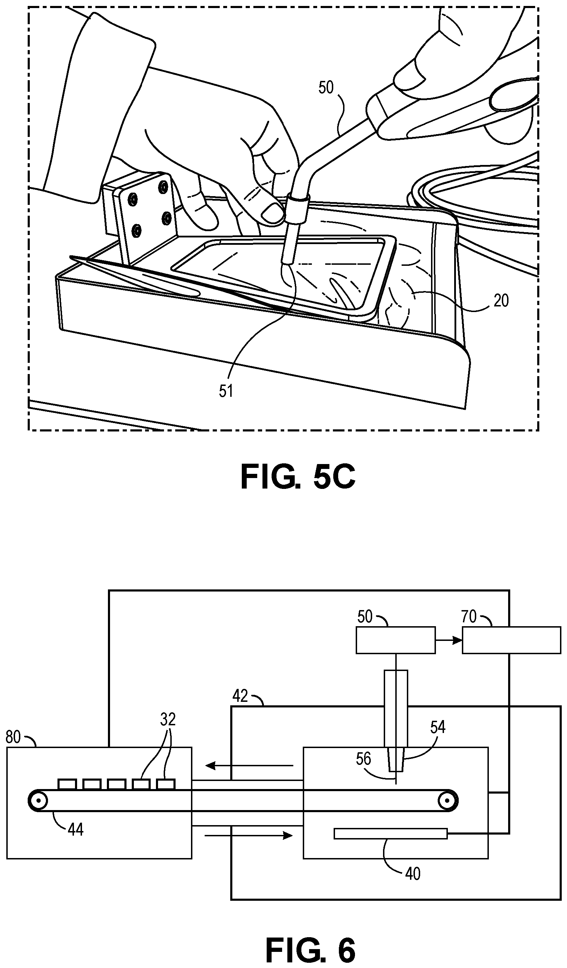

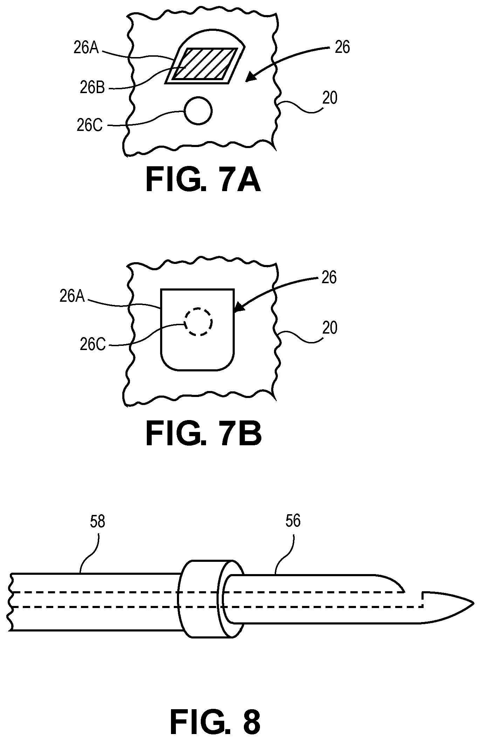

The one or more reaction chambers 20 each optionally include a hermetically sealable port 26 disposed in the gas impermeable material of the reaction chamber 20. The hermetically sealable port 26 is configured to allow fluid connection of the gas meter 50 with the inner volume 28. Fluid connection can occur by any suitable manner, such as by allowing controlled insertion of, or capping by, a fluid accepting orifice 51 of the gas meter 50 (e.g., as shown in FIGs 5B and 5C) or the connector device 56 (e.g., a needle or tip with an orifice), as described herein. The hermetically sealable port 26 can be a different opening from the opening 25, and may be disposed in a wall of reaction chamber 50 or in a lid for covering the opening 25. In an embodiment, the hermetically sealable port 26 can comprise a tab 26A of material with a layer of adhesive 26B disposed thereon. As shown in FIGS. 7A and 7B, the tab 26A can cover or uncover a perforation 26C in the reaction chambers 20. The perforation 26C can be included as part of the one or more reaction chamber 20 prior to the process of testing. Alternatively, the perforation 26C can be made in the reaction chamber during testing, such as when the gas measurement is carried out (e.g., a needle or other device can be used to puncture a wall of the reaction chamber), in which case the tab 26A is optional, although still useful for reducing gas leakage from the reaction chamber 20 after the perforation 26C is made, such as prior to and/or after gas concentration measurement. Alternatively, the port 26 can comprise a valve, such as a self-sealing valve, configured to allow access to the inner volume 28 of reaction chambers 20, such as a valve sized to allow a connector device 56 to be inserted. Suitable valves, such as self-sealing valves, are generally known. In another embodiment, the port 26 can be a septum, or septa, that covers an orifice of the reaction chamber 20, such as an orifice of vial, an orifice in the cap of a vial, an orifice in a bag, or an orifice in any of the other flexible or rigid reaction chambers described herein. The septum can comprise a polymer membrane, such as a PTFE or silicone membrane, that can prevent or reduce environmental contaminants and hermetically seal the port of the reaction chamber prior to measurement, while allowing for fluid connection by, for example, piercing of the septum with a connector device 56 (e.g., a needle). Septum-vials (sometimes referred to a septa-vials), as an example, are generally well known. A suitable septum for the port 26 can be chosen or designed by one of ordinary skill in the art. The type of port employed may depend on the type and material of the reaction chambers used and the type of connector device 56 to be employed. Choosing and/or designing suitable hermetically sealable ports, such as a tab for covering a perforation, a septum or a self-sealing valve, is well within the ordinary skill of the art. In an embodiment, other than the opening 25 and optional port 26, the one or more reaction chambers have no additional openings. Thus, when the opening 25 and optional port 26 are hermetically sealed, the reaction chambers can be airtight. Additional hermetically sealable openings can be included if desired.

In an embodiment, the system 10 can optionally include at least one reactant reservoir 30. The reactant reservoirs 30 can be a separate component that can be inserted into the reaction chambers 20. Different types of reactant reservoirs can be employed. In an embodiment, a reactant reservoir 30A can comprise a material or container that can hold a reactant for a time (e.g., prior to reaction) and then release all or a portion of the reactant at a later time, such as while the reaction chambers 20 are positioned in the enclosure 42. An example of a reactant reservoir 30A is a sponge or other porous material that can hold water or another reactant in the liquid phase and then release the reactant as it phase changes to a vapor as temperatures increase to the reaction temperature and/or as it is absorbed as a liquid when positioned in contact with an absorbent or porous material of the reservoir 30B. In an embodiment, a reactant reservoir 30B is a container configured to hold a solid phase reactant, such as a test product, and that is permeable to reactants in the vapor phase and/or liquid phase, such as a reactant contained in reservoir 30A. As an example, reservoir 30B can be a container comprising a vapor and/or liquid permeable material (e.g., an envelope or other packet comprising filter paper). Alternatively, one or both of reservoirs 30A and 30B can be a container capable of holding a reactant in solid or liquid form with a vapor permeable lid (e.g., a vial with a perforated lid). One or both of the reactant reservoirs 30A and 30B can be designed to be inserted into the one or more reaction chambers 20. In an embodiment, one or more of the reactant reservoirs 30A and 30B can be an integrated part of the reaction chambers 20 or the volume control insets 24. For example, pockets or other containers capable of functioning as reservoirs 30A and/or 30B can be integrated into the inner volume of the reaction chambers 20 or as part of the volume control insets 24.

Referring again to FIG. 2, the system 10 includes an energy source 40 suitable for driving a reaction in the one or more reaction chambers 20. The energy source 40 can provide thermal energy, radiation, or combinations thereof. Examples of suitable energy sources include heating elements; induction heaters; combustion (e.g., gas burners); radiation sources, such as lamps or lasers, that emit one or more of UV, IR, visible light or other wavelengths; magnetrons, or combinations of any of these energy sources.

As illustrated in FIG. 2, the energy source can optionally be positioned in an enclosure 42 that allows for a controlled reaction environment. The enclosure 42 can be suitably large to allow space for one or more, such as a plurality, of the reaction chambers 20 to be processed simultaneously in a controlled environment. For example, 2, 3 or more, such as 5 to 100 or more reaction chambers 20 can be simultaneously exposed to energy from the energy source 40 in the enclosure 42. In an embodiment, the enclosure 42 is a chamber with a thermal heat source, such as an oven. Alternatively, the energy source does not include an enclosure. For example, the energy source 40 can be a heating blanket or any other heat source capable of providing the desired reaction temperatures within the reaction chambers 20.

The system 10 further includes a gas meter 50 for measuring the concentration of a gas within the one or more reaction chambers 20. Any type of gas meter can be employed, such as a meter for measuring concentrations of hydrogen gas (H₂), oxygen gas (O₂), carbon monoxide, carbon dioxide, chlorine or other gases. The gas meter 50 can measure concentrations to any desired level of accuracy. For example, the gas meter 50 can be accurate within about ±200 ppm or better, about ±100 ppm or better, or about ±50 ppm or better. The accuracy of the gas meter can depend on many factors, including the range of gas concentrations being measured, and will generally be specified by the manufacturer of the gas meter. In an embodiment, the meter is a hydrogen gas meter. In an example, the gas meter can allow concentrations to be measured within a desired degree of accuracy over a limited range of concentrations of the gas being measured. For example, the gas meter may provide the desired accuracy (e.g., about ±200 ppm or better, ±100 ppm or better, or ±50 ppm or better) if the gas product being measured is within concentrations ranging from about 0 ppm to 5000 ppm, or from about 1 ppm to about 2000 ppm, or about 50 ppm to about 3000 ppm. The actual concentration range will vary depending on many factors, including the type of gas product being measured and the design of the gas meter 50. Suitable gas meters can be commercially available or later developed. An example of a commercially available gas meter is an INFICON Sensistor Sentrac Hydrogen Leak Detector, available from Inficon Holding AG of Bad Ragaz, Switzerland.

The gas meter 50 can be a hand held device that can be employed by a user to manually measure the gas concentration (FIG. 5C). In an embodiment, a handheld gas meter 50 can be connected to laptop 70, such as by a wired or wireless connection, to allow for uploading of concentration data, or the data can be input by the user, Alternatively, the gas meter 50 can be integrated as part of the system 10 for remotely and/or automatically measuring gas concentrations, as shown for example, in FIGS. 1, 5 and 6. Referring to FIG. 2, the gas meter 50 is indirectly connected to the inner volume 28 of each reaction chamber 20 using a conduit 58 that is fluidly connected to a connector device 56. The conduit 58 can be flexible so as to allow the connector device 56 to be positioned proximate to, and fluidly connected with, a reaction chamber 20, while the reaction chamber 20 is inside of enclosure 42. Alternatively, the conduit 58 and connector device 56 can be configured to be outside of the enclosure 42 instead of inside the enclosure. Configuring the conduit 58 and connector device 56 outside of the enclosure 42 can allow the one or more reactions chambers to be removed from the enclosure 42 prior to measuring the gas concentrations.

The connector device 56 can be any device that is configured to provide fluid connection to the inner volume 28. For example, the connector device 56 can be configured to be inserted into the reaction chamber 20, or otherwise attaching to or covering an orifice or a port 26 of, the one or more reaction chambers 20. In an example, the connector device 56 is a needle comprising an inner channel (as shown by the dotted lines in FIG. 8) capable of either being inserted through a wall of the reaction chambers 20 (e.g., by puncturing the wall), or of being inserted into the port 26 of the reaction chamber, thereby establishing the desired fluid connection and allowing gas from the reaction chamber 20 to flow to the gas meter 50. In another example, connector device 56 can comprise a tip with an orifice, similar to orifice 51 (FIG. 5) for capping, or covering, a port 26, such as a perforation 26C.

The system 10 can optionally further include any additional system components desired for establishing the fluid connection. For example, the system 10 can include a mechanical extender 54 attached directly or indirectly to the conduit 58 and/or the connector device 56. The mechanical extender 54 can be configured to position the connector device 56 to a position proximate the desired point of connection with a reaction chamber 20 and connecting the connector device 56 to the reaction chamber 20. Examples of suitable mechanical extenders 54 include telescoping and/or articulating arms. The mechanical extender 54 can further comprise one or more actuators, such as electrical, pneumatic or hydraulic actuators, to provide the desired force, torque and/or displacement for movement and control of the mechanical extender. The mechanical extender 54 can be automated, such as by employing a computer that has been programmed to control the device (e.g., computer 70 or another computer) or can be manually operated. An example of a suitable mechanical extender includes programmable robotic arms, which are well known in the art. One of ordinary skill in the art can readily choose and/or develop suitable mechanical extenders to position and attach the connector device 56 to establish fluid connection with each of the one or more reaction chambers 20.

Referring to FIG. 5A, system 10 can further comprise a fixture 32 configured to support at least one of the one or more reaction chambers 20. The fixture 32 can be designed to hold the reaction chambers 20 in position while the gas concentration is measured.

FIG. 5A illustrates one example of a fixture 32 that comprises a frame 34 and a base 35. A spring 36 is used to apply force to frame 34 to hold a reaction chamber 20 against the base 35. The reaction chamber 20 is placed in the base 35 with the middle section 24A of the volume control inset positioned adjacent the base 35 and the edges of the outer sections 24B positioned to extend upward from the middle section. Frame 34 can be configured to fit over the reaction chamber material and the volume control inset 24 positioned within the reaction chamber 20, proximate the perimeter of the volume control inset 24. This arrangement can maintain a portion of the flexible material of the reaction chamber 20 suspended over a pocket of gas between the edges of the outer sections of the volume control inset 24, as fluid connection between the inner volume of the reaction chamber 20 and the gas meter 50 is established. This can make it easier to establish the desired fluid connection (e.g., by allowing a needle or other connector device 56 to more easily perforate a wall of the reaction chamber 20, or to be inserted through a port 26, and into a pocket of gas in the inner volume 28). Various modifications can be made to the fixture 32, such as the shape of the frame 34 and/or the base 35. Further, while the frame 34 is spring-loaded as shown in FIG. 5A, any other suitable mechanism can be used in place of spring 34 to apply force to hold a reaction chamber 20 against the base 35. Furthermore, any mechanisms other than a frame 34 and base 35 design that are suitable for holding the reaction chambers 20 in position during gas measurement can be employed as fixture 32. Suitable designs for fixture 32 will vary depending on the shape and material of the one or more reactions chambers 20 and designing suitable fixtures 32 for a given reaction chamber design is within the ordinary skill of the art.

The fixture 32 can be used as a standalone device, such as for measuring gas concentrations by hand, as shown in FIG. 5C. Alternatively, one or more fixtures 32 can be integrated with systems 10 of FIGS. 1 and 5. For example, a plurality of fixtures 32 can be positioned in, or attached to, enclosure 42. Alternatively, as shown in FIG. 6, a plurality of fixtures 32 can be positioned on, or attached to, a conveyor 44 for moving the reaction chambers 20 to various positions within the system 10. The plurality of fixtures 32 can be attached to the enclosure 42 or the conveyor 44 in any suitable manner, such as by using mechanical fasteners, such as bolts or screws, by welding, or by any other manner.

The system 10 can further comprising an auto-sampler 80, as illustrated in FIG. 6. The auto-sampler 80 can be configured to convey the one or more reaction chambers 20 to a reaction site where the reaction is to occur (e.g., such as within enclosure 42 and/or proximate the energy source 40) and/or to position the reaction chambers within reach of the mechanical extender 54. In an embodiment, the auto-sampler 80 can comprise one or more fixtures 32, as described above, where each fixture 32 is configured to support a reaction chamber 20. The auto-sampler 80 can further include a transport system, such as a conveyor 44 (e.g., conveyor belt or other mechanism), for transporting the reaction chambers 20 attached to the fixtures 32 to and from the enclosure 42. In addition, or as an alternative, to the conveyor 44, the transport system can comprise a robotic system for conveying and/or positioning the reaction chambers 20. Auto-samplers are well known in the art, and one of ordinary skill in the art would be able to select and/or design an auto-sampler configured for use with the system 10 without undue experimentation.

The system 10 can further comprising a computer 70. Computer 70 can include a single central processing unit (CPU) or a plurality of networked CPUs, standalone CPUs, or both, which together perform the computer processing and control functions of system 10. For example, computer 10 is capable of performing one or more functions, such as collecting and storing data, including sample data input from a user and/or data collected from system 10, such as one or more of sample size, sample concentration, data regarding the ambient conditions employed, such as ambient temperature and length of time the reaction chambers are maintained at the ambient conditions, an expected dilution factor for each reaction chamber, and the measured gas concentrations from gas meter 50 for each reaction chamber. The dilution factor can optionally be included and indicates the expected amount of dilution in the reaction chamber that may be used to avoid having the product gas that is being measured (e.g., H2 gas) exceed any upper detection limit for the gas meter being used. For example, the dilution factor may depend on the sample size being tested (e.g., weight of pigment sample) and the expected volume of the reaction chamber after it is sealed, and may account for any differences between sample sizes or concentration differences between the samples employed in the different reaction chambers. The dilution factor for each reaction chamber can then be used to calculated a final, adjusted concentration from the measured gas concentrations that takes such differences into account so as to allow a more accurate comparison between the reaction chambers. The computer can optionally provide a desired output of the test results, including the measured gas concentrations from each reaction chamber and/or a report with any other information desired by the user.

In an embodiment, the computer 70 can be employed for calibrating the gas meter 50 prior to measuring gas concentrations from reaction chambers 20. The process for calibrating the instrument can vary depending on the gas meter used. In general, the calibration process can be automated by providing the computer 70 with computer readable code that carries out a pre-programmed calibration process of the gas meter and then tests the gas meter 50 for accuracy. As illustrated in FIG. 2, this process can include employing a gas source 60 of known concentration of the gas to be measured. For example, if H₂ gas is measured by the gas meter 50, then a hydrogen source of known concentration can be used. The gas source 60 of known concentration can be put in fluid connection with the gas meter 50 using, for example, a computer controlled valve 62 that is configured to control the flow of hydrogen gas from the gas source 60 to the gas meter 50 through a conduit 64. The calibration carried out by the computer 70 can include, for example, putting the gas meter 50 in a pre-programmed calibration mode and providing samples of the known gas concentration to the gas meter at appropriate times using the computer controlled valve 62 as the gas meter 50 performs a pre-programmed calibration process. The gas meter 50 can be programmed to carry out the pre-programmed calibration process by the gas meter manufacturer. Such pre-programmed calibration processes using a manually supplied source gas of known concentration are known in the art, as are gas meters that perform such processes. Once the pre-programmed calibration process as carried out by the gas meter 50 is complete, the computer 70 can automatically test the gas meter 50 by providing an additional flow from gas source 60 of the gas of known concentration to the gas meter 50 and instructing the gas meter 50 to measure the gas concentration. If the measured gas concentration is determined to be accurate based on the known concentration of the gas source 60, then the gas calibration process as carried out by computer 70 can be determined to be complete. Alternatively, if the measured gas concentration is determined to be inaccurate based on the known concentration, then the gas calibration process, including providing additional flows from gas source 60 to gas meter 50, can be repeated until the gas meter 50 provides an acceptably accurate measurement of the gas concentration from gas source 60. After calibration is determined to be complete by computer 70, the gas meter 50 can then optionally be employed to measure gas concentrations from the one or more gas reaction chambers 20, such as by using any of the methods and systems described herein. The gas calibration system is illustrated as part of the system shown in FIG. 2, but can be similarly included in any of the systems 10 of the present application for carrying out calibration of the gas meter 50.

The computer 70 can be hard wired or wirelessly connected to one or more components of the system 10, such as one or more of the gas meter 50, enclosure 42 (e.g., such as the energy source 40 and/or an ambient controller and/or sensors (not shown) employed for controlling the temperature of enclosure 42), and the mechanical extender 54, so as to provide for collection of data, such as measured gas concentrations, temperature and/or other conditions of enclosure 42. The computer 70 can optionally be employed to control various functions of the components of system 10, including, for example, the energy source 40 for controlling reaction energy within enclosure 42 and/or the operation of the mechanical extender 54 for collecting gas samples from the one or more reaction chambers 20. The computer 70 can include a non-transitory computer readable medium 72 that comprises computer readable instructions for collecting and storing the data transmitted from gas meter 50 for each sample collected from reaction chambers 20 and optionally storing any of the other data described herein. The computer readable medium 72 can also optionally include computer readable instructions for carrying out one or more of any of the functions of system 10 as described herein, such as calibrating the gas meter 50, providing a desired output of the test results and controlling one or more of any of the components of system 10, as described herein. For example, the non-transitory computer readable medium 72 can comprise computer readable instructions for collecting and storing a gas concentration transmitted from the gas meter for each reaction chamber 20 and providing an output (e.g., on a user readable monitor and/or printed hardcopy) associating the measured gas concentration with a test product contained in the reaction chamber from which it was collected. The output can be in any suitable form, and may include, for example, a list of a plurality of products tested and the associated measured gas concentration for each test product listed.

The present disclosure is also directed to a method for determining the concentration of a gas product in one or more reaction chambers and/or a method of testing a product. The method comprises hermetically sealing at least one first reactant (sometimes referred to herein as "exposure reactant" or "additional reactant") and a test product comprising a second reactant (sometimes referred to herein as a test product reactant) in a reaction chamber 20. The reaction chamber 20 is exposed to ambient conditions sufficient to cause the first reactant to react with the test product to produce a gaseous product if the second reactant is available for reaction with the first reactant (e.g., if the test product is not sufficiently passivated to prevent the first reactant from contacting the second reactant). The concentration of the gaseous product in the reaction chamber 20 is then determined, such as by using gas meter 50, as described herein.

In an embodiment, the test product is a pigment, such as a pigment comprising at least one metal, such as aluminum, iron, copper, magnesium, zinc, manganese, silver, platinum, palladium, tungsten, nickel, cobalt, niobium, chromium, tin, titanium and alloys of any of these or other metals as the test product reactant; or a pigment that comprise nonmetals, such as polymers or compounds of metal (e.g., metal oxides, metal nitrides or metal carbides) as the test product reactant. Other suitable test products include non-pigment test products, such as foils, coatings, filler powders, nanowires and catalyst, where the non-pigment test products comprise a metal or non-metal (e.g., any of the metals or non-metals listed herein for pigments) as the test product reactant. The term "metal" as used herein is defined to include both elemental metals (i.e., metals in their pure form) and metal alloys. Any of the above listed metals can be either in the form of an elemental metal or an alloy of the listed metal (e.g., elemental aluminum or aluminum alloy), where the listed metal is the predominant element of the alloy (e.g., greater than 50 % by weight, such 60 wt.%, 80 wt.%, 90 wt.%, 95 wt.%, 99 wt.% or more), with the remaining portion of the alloy including one or more additional elements chosen from metals and non-metals. The additional metals in the alloy can be any suitable metals, including alkali metals (e.g., lithium, sodium), alkaline earth metals, transition metals, or other metals, such as any of the metals listed herein. Non-metals, such as silicon or carbon, can be employed in the alloy in a relatively minor amount, such as less than 10 wt.%, less than 5 wt.%, less than 2 wt.%, less than 1 wt.% or less than 0.5 wt.%. For example, an "aluminum alloy" or "alloy of aluminum" is predominantly aluminum (e.g., in some cases 99 wt.% or more) with one or more additional elements chosen from metals and non-metals, such as any of the metals or non-metals described herein for using in alloys. Other examples of alloys include steel, brass, bronze, Inconel (Ni-Cr-Fe), stainless steels, Hastalloys (Ni-Mo-Fe; Ni-Mo-Fe-Cr; Ni-Si-Cu) and titanium-based alloys, such as titanium mixed with carbon (Ti/C), titanium mixed with tungsten (Ti/W), titanium mixed with niobium (Ti/Nb), and titanium mixed with silicon (Ti/Si), and combinations thereof.

The exposure reactant can be any desired reactant that can produce a gas when reacted with the test product reactant. Examples of exposure reactants include water and acids, such as HCl and H₂SO₄.

Optionally, a catalyst can also be included in the one or more reaction chambers 20. Examples of suitable catalysts include alkaline or acidic reagents (e.g., ammonia or hydrochloric acid). The catalyst can be included in the reaction chamber 20 in a reservoir 30 or in any other manner.

The gaseous product can be any gas that is the product of the reaction between the test product reactant and the exposure reactant. In an embodiment, the gaseous product can be a compound chosen from hydrogen gas (H₂), oxygen gas (O₂), carbon dioxide, carbon monoxide and chlorine gas. In an embodiment, the gaseous product is hydrogen gas.

As an example, the test product comprises a metal, such as elemental aluminum or aluminum alloy, or any of the other metals described herein for use as the test product reactant, (e.g., a pigment comprising aluminum), the at least one reactant comprises water in liquid form, vapor form, or combinations thereof, and the gaseous product that is measured is hydrogen gas. In another embodiment, the test product comprises a metal, such as magnesium, zinc or copper, or any of the other metals described herein for use as the test product reactant, (e.g., a pigment comprising magnesium, zinc or copper), the at least one reactant comprises an acid, such as HCl or H₂SO₄, and the gaseous product that is measured is hydrogen gas.

In an embodiment, the test product and the exposure reactant are each added to one or more reactant reservoirs 30, and then the one or more reactant reservoirs 30 are introduced into the reaction chamber 20. For example, the exposure reactant can be introduced into a reactant reservoir 30A and the test product can be introduced into a reactant reservoir 30B. Any of the reactant reservoirs 30A and 30B described herein can be employed. Alternatively, one or both of the test product and exposure reactant can be introduced directly into the reaction chamber 20, either without use of a reservoir 30 or into reservoirs 30 that are integrated into the reaction chamber 20 itself.

The test product sample size in each reaction chamber 20 can be kept constant between a plurality of samples (e.g., the same, or substantially the same, weight of pigment can be used for each of a plurality of reaction chambers 20). This can allow for a more direct comparison of the measured gas product concentrations between the plurality of test product samples of the different reaction chambers 20, where the plurality can be, for example, a batch of test product samples as described below. For a similar reason, the amount of the one or more other reactants (e.g., exposure reactant) employed can be kept constant between reaction chambers 20 (e.g., the same, or substantially the same, weight of each reactant can be used for each reaction chamber 20). Substantially the same weight, as used herein, is taken to mean that weights between any two test product samples will vary by 10% or less, in the case of the sample size, or that weights of the other reactants employed in any two reaction chambers 20, will vary by 10% or less, in the case of the other reactants.

In an embodiment, the one or more reaction chambers 20 each have a variable inner volume. For example, the reaction chambers 20 can comprise a metalized bag or any other variable volume container described herein. Reaction chambers 20 having a variable volume can have advantages, such as being disposable, taking up little storage space and/or are relatively inexpensive. The method can further include inserting a volume control inset 24 into each of a plurality (e.g., a batch as described below) of the one or more variable volume reaction chambers 20 so as to provide each container with a volume that is a relatively more fixed volume as compared to the potential change in volume without the inset 24. In an embodiment, the volume control insets 24 employed in each of the plurality of the one or more reaction chambers 20 have the same shape, as well as corresponding dimensions that are substantially the same size, where "substantially the same size" is taken to mean that the corresponding length, width and height dimensions that can affect the resulting volume of the reaction chamber vary by 10% or less between any two insets. This can provide a relatively more constant volume between the one or more reactors than if the corresponding dimensions of the insets were to vary by a larger amount (e.g., by 25% or more). In an embodiment, the variable volume reactors with the volume control insets 24 can have approximately the same volume after being hermetically sealed, where "approximately the same volume" is taken to mean that any two reactors of the plurality have volumes that differ by 20% or less. In other examples, the volumes may vary by 15% or less, or 10% or less. This can allow the concentration of the gas product measured for each test product to be directly compared with the concentrations of gas product measured for the other test products without having to account for volume differences between the chambers. Alternatively, the one or more reaction chambers 20 have a fixed volume and no volume control inset need be employed.

The one or more reaction chambers 20 are hermetically sealed by any suitable method. For containers with a variable volume, such as a bag, this can comprise applying heat and/or pressure to a portion of the reaction chamber proximate the opening 25 to fuse the material of the reaction chamber 20 together and thereby form a seal that effectively closes the opening. Other techniques that can supply a hermetic seal that will withstand reaction temperatures, such as, for example, use of an adhesive or a clamp to seal the opening 25, can also be employed. If the one or more reaction chambers 20 comprise a container with a hermetically sealable lid, the lid can simply be positioned onto the container to provide the desired seal.

The sealed reaction chambers 20 are exposed to ambient conditions sufficient to cause the exposure reactant to react with the test product reactant to react to produce a gaseous product if the test product reactant is available for reaction with the exposure reactant (e.g., if the test product is not sufficiently passivated to prevent the exposure reactant from contacting the test product reactant). In an embodiment, this can include inserting the one or more reaction chambers 20 into an enclosure 42 heated to a reaction temperature suitable for carrying out the reaction. As examples, reaction temperatures can range from about 35 °C to about 500 °C, such as about 50 °C to about 300 °C or about 50 °C to about 80 °C. Alternatively, or in addition to heating, the reaction chambers 20 can be exposed to any of the other energy sources described herein to drive the reaction.

The reaction chambers 20 can be maintained at ambient conditions sufficient to cause reaction for any suitable time period. Examples of suitable time periods can be about 1 week or less, such as 1 hour to about 72 hours, or about 6 hours to about 48 hours, or about 12 hours to about 36 hours, or about 24 hours.

The concentration of the gaseous product in the one or more reaction chambers 20 is then determined, such as by using a gas meter 50, such as any of the gas meters described herein. In an embodiment, the reaction chambers 20 are removed from the ambient conditions prior to determining the concentration. For example, the reaction chambers 20 can be removed from the enclosure 42 and optionally allowed to cool prior to measuring the concentration. Alternatively, the gas concentration can be determined at the ambient conditions, such as, for example, inside enclosure 42.

Determining the gas concentration of the product gas comprises fluidly connecting the gas meter 50 with an inner volume 28 of the one or more reaction chambers 20. This can be accomplished using any of the devices described herein, such as the connector device 56 and optionally the mechanical extender 54, as shown in FIG. 2. For example, where the connector device is a needle, the needle may be used to puncture the reaction chamber walls or to be inserted into a port 26 in the reaction chambers 20, either by hand or by employing the mechanical extender 54. In an alternative embodiment, a user can insert a tip of the gas meter 50 into either a perforation made by the user or into a preexisting port 26 in the reaction chambers 20. Measuring the gas concentration can be carried out on a reaction chamber 20 either before, or after, the reaction chamber has been removed from the ambient conditions used to drive the reaction (e.g., before or after the reaction chamber is removed from enclosure 42). Optionally, a fixture 32, such as any of the fixtures 32 described herein, can be employed to hold at least one of the one or more reaction chambers 20 when the gas concentration is measured. The measured gas product concentrations can be recorded by a user or automatically input as data into the computer 70, such as when using the system 10 of FIG. 2.

The method can be carried out for one or a plurality of test products. In an embodiment, a plurality of test products are tested, the plurality of products making up a test batch. The test batch can comprise any number of a plurality of test products, such as 2 to 1000 test products or more, each test product being introduced into a separate reaction chamber 20. For example, the number of test products can range from 2 to 500, 3 to 100, or 5 to 50, as examples. A corresponding number of reaction chambers 20 is employed, one for each test product of the test batch.

The test products in the test batch can include the same and/or different test products. For example, test products can include a second test product that is the same as a first test product (e.g., a different sample of the same product comprising the same material); and/or the test products can include a second test product that is a different product from the first test product (e.g., a product comprising a different material or different concentrations of materials). In an embodiment, at least some of the test products are different from other test products in the same batch.

In an embodiment, most (e.g., more than 50%, such as more than 90%) or all of the test products of a test batch comprise a test product reactant that can react with the same exposure reactant to form the same gaseous product. For example, all of the test products of a test batch can include a test product reactant that comprises a metal element as a common reactant element, where the metal element can be included in at least one form, such as the metal in elemental form (e.g., the pure metal), an alloy of the metal element or a compound of the metal element, in each of the test products. Thus, all test products in the test batch can comprise a common reactant element, such as aluminum, where the common reactant element in each test product can be included as part of a same material in each of the test products, or alternatively as part of different materials (e.g., an elemental metal, such as elemental aluminum, or different alloys of that same metal, such as different aluminum alloys) can be used in some or all of the test products in the batch). Examples of suitable common reactant metal elements that can be employed in each test product in a batch include aluminum, iron, copper, magnesium, zinc, manganese, silver, platinum, palladium, tungsten, nickel, cobalt, niobium, chromium, tin or titanium. Alternatively, test products having a common reactant non-metal element can also be used, such as where carbon is the common non-metal element when testing different polymers using the systems and methods of the present disclosure. Alternatively, the test products of a test batch comprise a test product reactant that can react with the same exposure reactant to form a same gaseous product, where the test product reactants do not include a common reactant element.

The same or different exposure reactant, such as any of the exposure reactants described herein, can be employed in each of a plurality of the reaction chambers 20. In an embodiment the same exposure reactant is employed in each of a plurality (e.g., a batch as described herein) of the reaction chambers 20.

In an embodiment, the average ambient temperature (e.g., the temperature of enclosure 42) over the time period the reaction chambers are exposed to the energy source can be the same or substantially the same for each of a plurality (e.g., a batch as described herein) of the reaction chambers 20, where the reaction chambers are exposed for the same, or substantially the same, period of time. The average ambient temperature can be, for example, within any of the range of reaction temperatures described herein. "Substantially the same", as used with respect to the average ambient temperature or the period of time, is taken to mean that the percentage difference between average ambient temperatures or the period of time, respectively, for any two reaction chambers vary by 10% or less. As further examples, the percentage difference between average ambient temperatures or period of time for any two reaction chambers can vary by less than 5%, or less than 2%. As used throughout this application, "substantially the same" is considered to encompass "the same". Thus, two reaction chambers that are exposed to "the same" average ambient temperature would also be considered to be exposed to "substantially the same" average ambient temperature.

In an example, the method can comprise hermetically sealing a first test product and a first exposure reactant in a first reaction chamber 20 and hermetically sealing a second test product and a second exposure reactant in a second reaction chamber 20. The first test product is different than the second test product and both the first test product and the second test product comprise a test product reactant (e.g., the different pigments each comprise aluminum, either as elemental aluminum or an aluminum alloy, and/or they are the same pigments with different passivation layers encapsulating the pigments). The first exposure reactant and the second exposure reactant are the same (e.g., water is included as the exposure reactant in both reactant chambers). The method can comprise exposing both the first reaction chamber and the second reaction chamber to the same, or substantially the same, average ambient temperature, or other reaction energy, for the same, or substantially the same, period of time, which is sufficient to cause the exposure reactant to react with the test product reactant to produce a gaseous product if the test product reactants are available for reaction, as described herein. A concentration of the gaseous product is then determined in both the first reaction chamber and in the second reaction chamber. As described above, the method can be carried out for any number of test products, such as a batch of test products as described herein.

The process can optionally include employing the measured concentration data of the gaseous product for each of the test products to determine whether or not one or more of the different test products are suitable for a chosen application. For example, the process can include determining which of the test products produced a gas concentration, C_{g}, of the gaseous product that is within an acceptable range and optionally generating a list of the acceptable test products that meet this condition. If desired, the concentration data for the test products can be compared to determine which test product or products provide the most desired outcomes (e.g., the lowest gas concentration values).

The present disclosure is also directed to a test kit having one or more components of system 10. The system comprises various elements that can be changed and adjusted to provide desired test conditions, reduce testing time, increase the precision and/or accuracy of the test results and/or repeatability of the test function. The kit comprises one or more reaction chambers 20. Each reaction chamber comprises (i) a gas impermeable material enclosing an inner volume and (ii) a hermetically sealable opening, as described herein. Any of the reaction chambers 20, as described herein, can be employed. The test kit can further comprise at least one reactant reservoir 30 configured to be inserted into the one or more reaction chambers 20, such as any of the reactant reservoirs described herein. Optionally, at least one reactant reservoir 30, such as any of the reactant chambers described herein, can be designed to function as a product container configured to hold a test product and to be inserted into the one or more reaction chambers. Optionally, the test kit can further include a gas meter capable of measuring a concentration of a gas in the one or more reaction chambers 20, such as any of the gas meters 50 described herein. Optionally, the test kit can further comprise an energy source 40, such as any of the energy sources 40 described herein. Optionally, the test kit can further comprise a volume control inset 24 configured to be inserted into each of the one or more reaction chambers 20, such as any of the volume control insets 24 described herein. Optionally, the test kit can comprise a fixture 32 configured to hold at least one of the one or more reaction chambers, such as any of the fixtures 32 described herein. Any of the other components described herein as part of system 10 can also optionally be included in the test kit. The components of the test kit can be packaged and/or sold together and may be employed to carry out any of the methods of the present disclosure.

### EXAMPLE

Pigment samples comprising aluminum were rinsed with isopropyl alcohol or another solvent and dried. The pigment samples were enclosed in coffee filter envelopes to keep the pigment contained (sometimes referred to as "pigment packets") and to allow the pigment packets to absorb moisture (e.g., in liquid and/or vapor form) during the test. Once prepared, the pigment samples remained in the envelope throughout the test. Pigments were weighed in filter envelopes.

ULINE^{™} Dri-Shield Bags (referred to as "sample bags") were used to pre-condition and test samples, as described below. A polycarbonate inset (e.g., shaped as in FIG. 4A) was inserted into each sample bag, with the inset's folded corners positioned toward bottom of the bag and the inset's flat side facing the back of bag (see FIG. 4B). The pigment in the filter envelopes was positioned inside the bag in a pocket formed by the inset. A plurality of the sample bags (one for each pigment sample) were set in a baking pan with open ends standing up. The baking pan with samples were placed in an 80°C oven and the temperature was monitored for 1 hour to precondition the pigment samples.

Sponges were rinsed to remove contaminants and baked in an oven to dry for pre-conditioning. The sponges were pre-conditioned immediately before being added to the sample bags by including the sponges in the oven alongside the pre-conditioning pigment samples. Following preconditioning, 1.00g ± 0.01g of DI water was added dropwise to each sponge. The water was distributed so that the entire top surface of the sponge was wet. The weight of each pigment sample and water weight for each sponge were recorded.

A wet sponge was added to each sample bag alongside a pigment packet so that they are side-by-side. The sample bags were straightened out so that the bottom of the bag was not collapsed against the polycarbonate shape by pulling at bottom corners. This can help insure that volumes inside all bags are approximately equal so that measured hydrogen concentrations are comparable between the samples. The sample bags were then sealed using a HIPPO heat sealer. The sample bags were placed in a baking pan, which was then placed in oven at about 63 °C for about 24 hours.

The sample bags were removed from the oven and allowed to cool. Each sample bag was then placed into a sample holding fixture (e.g., such as shown in FIG. 5C). While positioned in the holding fixture, the bag was perforated to make a small hole, which was immediately covered with aluminized PET tape to reduce gas leakage. The tape was partially pulled away from the bag to expose the hole. The tip of a calibrated INFICON hydrogen leak detector was immediately placed against the bag over the hole and the bag was compressed while the concentration of hydrogen measured.

In an aspect, the present disclosure is directed to a method for determining a concentration of a gaseous product in one or more reaction chambers, the method comprising: hermetically sealing an exposure reactant and a test product comprising a test product reactant in one or more reaction chambers; exposing the one or more reaction chambers to ambient conditions sufficient to cause the exposure reactant and the test product reactant to react to form a gaseous product if the test product reactant is available for reaction with the exposure reactant; and determining the concentration of the gaseous product in the one or more reaction chambers. In an aspect, the test product reactant is a metal chosen from aluminum, iron, copper, magnesium, zinc, manganese, silver, platinum, palladium, tungsten, nickel, cobalt, niobium, chromium, tin, titanium and alloys thereof; and the exposure reactant is chosen from water and an acid. In an aspect, the gaseous product is a compound chosen from hydrogen gas, oxygen gas, carbon dioxide, carbon monoxide and chlorine gas. In an aspect, the test product is a pigment, a foil, a coating, a filler powder, a nanowire or a catalyst. In an aspect, the method further comprises carrying out the processes of hermetically sealing, exposing the reaction chamber and determining the concentration of the gaseous product for a plurality of test products in two or more reaction chambers, where each test product is contained in a separate reaction chamber of the two or more reaction chambers. In an aspect, the exposing of the one or more reaction chambers to ambient conditions sufficient to cause the exposure reactant and the test product reactant to react is carried out for a time period that ranges from about 1 hour to about 1 week. In an aspect, the one or more reaction chambers each have a variable volume. In an aspect, the method further comprises inserting a volume control inset into each of the one or more reaction chambers. In an aspect, the one or more reaction chambers each comprise a metalized bag. In an aspect, the method further comprises inserting at least one reactant reservoir into the one or more reaction chambers, wherein the at least one reactant reservoir is gas permeable.

In an aspect, the present application is directed to a test kit having two or more components, the kit comprising: one or more reaction chambers, each reaction chamber comprising (i) a gas impermeable material enclosing an inner volume and (ii) a hermetically sealable opening; at least one reactant reservoir configured to be inserted into the one or more reaction chambers; and a volume control inset configured to be inserted into each of the one or more reaction chambers so as to provide each container with a desired volume. In an aspect, the test kit further comprises a gas meter capable of measuring a concentration of a gas in the one or more reaction chambers; and optionally a fixture configured to hold the one or more reaction chambers while measuring the concentration of gas.

An aspect of the present disclosure is also directed to a reaction chamber, comprising: a gas impermeable material at least partially enclosing an inner volume; a hermetically sealable opening; and a hermetically sealable port disposed in the gas impermeable material, wherein the reaction chamber has a variable inner volume.

From the foregoing description, those skilled in the art can appreciate that the present teachings can be implemented in a variety of forms. Therefore, while these teachings have been described in connection with particular embodiments and examples thereof, the true scope of the present teachings should not be so limited. Various changes and modifications can be made without departing from the scope of the teachings herein.

This scope disclosure is to be broadly construed. It is intended that this disclosure disclose equivalents, means, systems and methods to achieve the devices, activities and mechanical actions disclosed herein. For each device, article, method, mean, mechanical element or mechanism disclosed, it is intended that this disclosure also encompass in its disclosure and teaches equivalents, means, systems and methods for practicing the many aspects, mechanisms and devices disclosed herein. The claims of this application are likewise to be broadly construed. The description of the inventions herein in their many embodiments is merely exemplary in nature and, thus, variations that do not depart from the gist of the invention are intended to be within the scope of the invention. Such variations are not to be regarded as a departure from the spirit and scope of the invention.

## Claims

1. A system for determining a concentration of a gas in one or more reaction chambers, the system comprising:
an energy source configured to provide a reaction energy to the one or more reaction chambers; and
a gas meter capable of measuring a concentration of a gas in the one or more reaction chambers.

2. The system of claim 1, wherein the gas meter is a hydrogen gas meter, oxygen gas meter, carbon monoxide gas meter, carbon dioxide gas meter or a chlorine gas meter.

3. The system of claim 1 or claim 2, further comprising a fixture configured to hold at least one of the one or more reaction chambers.

4. The system of any of claims 1 to 3, further comprising an auto-sampler configured to convey the one or more reaction chambers to a reaction site.

5. The system of any of claims 1 to 4, further comprising a connector device fluidly connected to the gas meter, the connector device configured to fluidly connect the gas meter with an inner volume of the one or more reaction chambers.

6. The system of any of claims 1 to 5, further comprising a computer that includes a non-transitory computer readable medium, the non-transitory computer readable medium comprising computer readable instructions for collecting and storing a gas concentration transmitted from the gas meter for each reaction chamber tested and providing an output associating the gas concentration with a sample contained in the reaction chamber from which it was collected.

7. A method for determining a concentration of a gaseous product in one or more reaction chambers, the method comprising:
hermetically sealing an exposure reactant and a test product comprising a test product reactant in one or more reaction chambers;
exposing the one or more reaction chambers to ambient conditions sufficient to cause the exposure reactant and the test product reactant to react to form a gaseous product if the test product reactant is available for reaction with the exposure reactant; and
determining the concentration of the gaseous product in the one or more reaction chambers.

8. The method of claim 7, wherein the test product reactant is a metal chosen from aluminum, iron, copper, magnesium, zinc, manganese, silver, platinum, palladium, tungsten, nickel, cobalt, niobium, chromium, tin, titanium and alloys thereof; and wherein the exposure reactant is chosen from water and an acid.

9. The method of claim 7 or claim 8, wherein the gaseous product is a compound chosen from hydrogen gas, oxygen gas, carbon dioxide, carbon monoxide and chlorine gas.

10. The method of any of claims 7 to 9, wherein the test product is a pigment, a foil, a coating, a filler powder, a nanowire or a catalyst.

11. The method of any of claims 7 to 10, further comprising carrying out the processes of hermetically sealing, exposing the reaction chamber and determining the concentration of the gaseous product for a plurality of test products in two or more reaction chambers, where each test product is contained in a separate reaction chamber of the two or more reaction chambers.

12. The method of any of claims 7 to 11, wherein the one or more reaction chambers each have a variable volume; and wherein the method further comprises inserting a volume control inset into each of the one or more reaction chambers.

13. The method of any of claims 7 to 12, wherein the one or more reaction chambers each comprise a metalized bag.

14. A test kit having two or more components for use with the system of any of claims 1 to 6, the kit comprising:
one or more reaction chambers, each reaction chamber comprising (i) a gas impermeable material enclosing an inner volume and (ii) a hermetically sealable opening;
at least one reactant reservoir configured to be inserted into the one or more reaction chambers; and
a volume control inset configured to be inserted into each of the one or more reaction chambers so as to provide each container with a desired volume.

15. A reaction chamber for use with the system of any of claims 1 to 6, comprising:
a gas impermeable material at least partially enclosing an inner volume;
a hermetically sealable opening; and
a hermetically sealable port disposed in the gas impermeable material,
wherein the reaction chamber has a variable inner volume.
